# EUROPEAN PATENT APPLICATION

(11) **EP 4 644 414 A1**
(43) Date of publication of application: **05.11.2025**
(21) Application number: 23910552.1
(22) Date of filing: 26.12.2023
(51) Int. Cl.: C07K 16/08, C12N 15/13, C12N 15/62, G01N 33/569

(54) **MONOCLONAL ANTIBODY AGAINST CAPSID PROTEINS OF MULTIPLE AAV SEROTYPES, PREPARATION METHOD THEREFOR, AND USE THEREOF**

(30) Priority: 26.12.2022 CN 202211686491
(71) Applicant: Nanjing GenScript Biotech Co., Ltd., Nanjing, Jiangsu 211122 (CN)
(72) Inventor: DING, Lidan, Nanjing, Jiangsu 211122 (CN); WANG, Fei, Nanjing, Jiangsu 211122 (CN); HE, Yuzhuo, Nanjing, Jiangsu 211122 (CN); LI, Jingqi, Nanjing, Jiangsu 211122 (CN)
(74) Representative: Blumbach · Zinngrebe Patentanwälte PartG mbB
(86) International application number: PCT/CN2023/141746
(87) International publication number: WO 2024/140627

(57) **Abstract**

The present invention belongs to the field of virus detection and diagnosis, and relates to a monoclonal antibody against capsid proteins of multiple AAV serotypes, a preparation method therefor, and the use thereof. Provided in the present invention are a rabbit monoclonal antibody against capsid proteins of multiple AAV serotypes, and amino acid sequences of a heavy-chain variable region and a light-chain variable region of same. Said rabbit monoclonal antibody provided by the present invention can specifically bind to capsid proteins VP1, VP2 and VP3 of multiple AAV serotypes, and thus can be used for detecting AAV antigens. The monoclonal antibody against capsid proteins of multiple AAV serotypes provided by the present invention enables and facilitates research and development processes of gene therapy vectors and detection of AAVs in basic scientific research.

## Description

### Cross-Reference to Related Applications

The present application claims priority to Chinese Patent Application No. 202211686491.0, filed on December 26, 2022, which is incorporated herein by reference in its entirety.

### Technical Field

The present invention belongs to the field of virus detection and diagnosis, and relates to a monoclonal antibody against capsid proteins of multiple AAV serotypes. The present invention also relates to a method for preparing the monoclonal antibody against capsid proteins of multiple serotypes and the use of the monoclonal antibody against capsid proteins of multiple serotypes.

### Background Art

Adeno-associated virus (AAV) belongs to the family Parvoviridae and is a non-enveloped single-stranded linear DNA virus. The genome of AAV is about 4.7 kb in length and mainly includes two genes, Rep and Cap. The Cap gene encodes three viral capsid proteins, VP1, VP2 and VP3. The C-terminal sequences of the VP1 and VP2 proteins are identical to the sequence of the VP3 protein. In the viral capsid, VP3 has the highest content, while the contents of VP1 and VP2 are each only one-tenth of that of VP3.

The AAV virus is a non-pathogenic virus that can infect humans and a variety of other vertebrates. There are many serotypes of AAV, including AAV1, AAV2, AAV3, AAV4, AAV5, AAV6, AAV7, AAV8, AAV9, AAVDJ, AAVrh10, etc. Due to the low immunogenicity, and the fact that different serotypes of AAVs have different targeting properties and expression efficiencies for different tissues and organs, AAV has become a commonly used genetic manipulation tool and plays a key role in both active immunotherapy by delivering antigens and passive immunotherapy by delivering antibodies. The AAV virus can also cross the blood-brain barrier, providing a new method for the treatment of central nervous system diseases. The CRISPR-Cas9 system carried by the AAV virus also provides a new means for gene editing in organ tissues such as the brain, cochlea and muscle.

Due to the large number of AAV virus serotypes, newly discovered and newly developed AAV vectors are also emerging. The monoclonal antibody against capsid proteins of multiple AAV serotypes is a convenient detection tool in the research and development processes of AAV gene therapy vectors.

### Summary of the Invention

In one aspect, the present invention provides a monoclonal antibody against capsid proteins of multiple AAV serotypes or a functional fragment thereof, wherein the antibody or the functional fragment thereof comprises heavy chain CDRs and light chain CDRs, the heavy chain CDRs and light chain CDRs are selected from the group consisting of CDRs with amino acid sequences as set forth in SEQ ID NO: 8 to SEQ ID NO: 19 and variants thereof each comprising at most three amino acid mutations, and the group is defined in terms of a combination pattern by a and/or b:

| Combination name | Heavy chain CDR1 | Heavy chain CDR2 | Heavy chain CDR3 | Light chain CDR1 | Light chain CDR2 | Light chain CDR3 |
|---|---|---|---|---|---|---|
| a | SEQ ID NO: 8 or a variant thereof | SEQ ID NO:9 or a variant thereof | SEQ ID NO:10 or a variant thereof | SEQ ID NO:11 or a variant thereof | SEQ ID NO: 12 or a variant thereof | SEQ ID NO: 13 or a variant thereof |
| b | SEQ ID NO: 14 or a variant thereof | SEQ ID NO: 15 or a variant thereof | SEQ ID NO: 16 or a variant thereof | SEQ ID NO: 17 or a variant thereof | SEQ ID NO: 18 or a variant thereof | SEQ ID NO: 19 or a variant thereof |

In some embodiments, the heavy chain CDRs and light chain CDRs are selected from the group consisting of CDRs with amino acid sequences as set forth in SEQ ID NO: 8 to SEQ ID NO: 19.

In some embodiments, the heavy chain CDRs and light chain CDRs are selected from the following sequences:
a. heavy chain CDR1, heavy chain CDR2, heavy chain CDR3, light chain CDR1, light chain CDR2, and light chain CDR3 with amino acid sequences as set forth in SEQ ID NOs: 8, 9, 10, 11, 12 and 13, respectively; or
b. heavy chain CDR1, heavy chain CDR2, heavy chain CDR3, light chain CDR1, light chain CDR2, and light chain CDR3 with amino acid sequences as set forth in SEQ ID NOs: 14, 15, 16, 17, 18 and 19, respectively. In some embodiments, the monoclonal antibody against capsid proteins of multiple AAV serotypes or the functional fragment thereof comprises a heavy chain variable region and a light chain variable region, wherein the heavy chain variable region and light chain variable region are selected from the group consisting of variable regions with amino acid sequences as set forth in SEQ ID NO: 4 to SEQ ID NO: 7 and variants thereof each comprising an amino acid sequence having at least 80% identity thereto, and the group is defined in terms of a combination pattern by A and/or B:

| Combination name | Heavy chain variable region | Light chain variable region |
|---|---|---|
| A | SEQ ID NO: 4 or a variant thereof | SEQ ID NO: 5 or a variant thereof |
| B | SEQ ID NO: 6 or a variant thereof | SEQ ID NO: 7 or a variant thereof |

In some embodiments, the heavy chain variable region sequence comprises an amino acid sequence having at least 80% identity to an amino acid sequence as set forth in SEQ ID NO: 4 or 6; and the light chain variable region sequence comprises an amino acid sequence having at least 80% identity to an amino acid sequence as set forth in SEQ ID NO:5 or 7. In some embodiments, the heavy chain variable region sequence comprises an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to an amino acid sequence as set forth in SEQ ID NO: 4 or 6; and the light chain variable region sequence comprises an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to an amino acid sequence as set forth in SEQ ID NO: 5 or 7.

In some embodiments, the heavy chain variable region and light chain variable region are selected from the following sequences:
(A) the heavy chain variable region comprising an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to a sequence as set forth in SEQ ID NO: 4, and the light chain variable region comprising an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to a sequence as set forth in SEQ ID NO: 5; or
(B) the heavy chain variable region comprising an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to a sequence as set forth in SEQ ID NO: 6, and the light chain variable region comprising an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to a sequence as set forth in SEQ ID NO: 7.

In some embodiments, the heavy chain variable region sequence comprises an amino acid sequence as set forth in SEQ ID NO: 4 or 6; and the light chain variable region sequence comprises an amino acid sequence as set forth in SEQ ID NO: 5 or 7.

In some embodiments, the heavy chain variable region and light chain variable region are selected from the following sequences:
A. the heavy chain variable region comprising an amino acid sequence as set forth in SEQ ID NO: 4 and the light chain variable region comprising an amino acid sequence as set forth in SEQ ID NO: 5; or
B. the heavy chain variable region comprising an amino acid sequence as set forth in SEQ ID NO: 6 and the light chain variable region comprising an amino acid sequence as set forth in SEQ ID NO: 7.

In some specific embodiments, the heavy chain variable region and light chain variable region are selected from the following sequences:
A. the heavy chain variable region with an amino acid sequence as set forth in SEQ ID NO: 4 and the light chain variable region with an amino acid sequence as set forth in SEQ ID NO: 5; or
B. the heavy chain variable region with an amino acid sequence as set forth in SEQ ID NO: 6 and the light chain variable region with an amino acid sequence as set forth in SEQ ID NO: 7.

In some embodiments, the antibody has a constant region, wherein the heavy chain constant region sequence is selected from a constant region sequence of any one of IgG1, IgG2, IgG3, IgG4, IgA, IgM, IgE and IgD; and the light chain constant region is a κ or λ chain.

In some embodiments, the monoclonal antibody against capsid proteins of multiple AAV serotypes or the functional fragment thereof is a rabbit-derived antibody, a chimeric antibody, a humanized antibody or a human antibody.

"Rabbit-derived antibody" refers to an antibody whose variable and constant regions (if present) are derived from rabbit immunoglobulin sequences. The rabbit antibody can be conveniently obtained by immunizing a rabbit with the corresponding antigen and isolating the target antibody therefrom. Alternatively, the rabbit antibody can be obtained by immunizing the rabbit with the corresponding antigen, and then isolating and culturing cells (such as B cells) expressing the target antibody. Alternatively, the target antibody (e.g. the monoclonal antibody) can be obtained in large quantities and for a long time by means of immunizing the rabbit with the corresponding antigen, then isolating and culturing cells expressing the target antibody, and fusing the cells with immortalized cells, such as myeloma cells, to obtain hybridoma cells, and culturing the hybridoma cells.

The term "chimeric antibody" is an antibody formed by fusing the variable region of a first animal-derived antibody to the constant region of a second animal-derived antibody. A chimeric antibody is established by means of firstly establishing a hybridoma that secretes a first animal-derived specific monoclonal antibody, then cloning a variable region gene from the hybridoma cell, cloning a constant region gene of a second animal-derived antibody as needed, linking the first animal-derived variable region gene and the second animal-derived constant region gene to form a chimeric gene, inserting the chimeric gene into an expression vector, and finally expressing a chimeric antibody molecule in an eukaryotic or prokaryotic system. In a preferred embodiment of the present invention, the first animal source is rabbit source, and the second animal source is preferably human source, which can relieve the immune response induced by the first animal-derived antibody. The antibody light chain of the chimeric antibody further comprises a light chain constant region of a human κ or λ chain or a variant thereof. The antibody heavy chain of the chimeric antibody further comprises a heavy chain constant region of human IgG1, IgG2, IgG3 or IgG4 or a variant thereof.

The term "humanized antibody", also known as a CDR-grafted antibody, refers to an antibody produced by grafting a first animal-derived CDR sequence into a human antibody variable region framework, i.e., different types of human germline antibody framework sequences. Such antibody can overcome the heterogeneous response induced by the chimeric antibody due to carrying a large amount of rabbit protein components. Such framework sequences can be obtained from public DNA databases or published references that include germline antibody gene sequences. For example, germline DNA sequences of human heavy and light chain variable region genes can be obtained in the "VBase" human germline sequence database (www.mrccpe.com.ac.uk/vbase), and found in Kabat, E. A. et al., 1991, Sequences of Proteins of Immunological Interest, 5th edition. In order to avoid a decrease in activity caused by a decrease in immunogenicity, the human antibody variable region framework sequence can be subjected to minimal reverse mutation or back mutation to maintain activity.

The humanized antibody of the present invention, when the degree of humanization thereof is further improved, also includes a humanized antibody (human antibody) obtained after further affinity maturation of CDRs by phage display. In a preferred embodiment of the present invention, the first animal source is rabbit source. The human antibody variable region framework is designed and selected, for example, the heavy chain FR region sequence on the antibody heavy chain variable region is derived from a combined sequence of human germline heavy chain IGHV1-18*01 and hjh6.1, or a combined sequence of human germline heavy chain IGHV1-3*01 and hjh6.1; and the light chain FR region sequence on the antibody light chain variable region is derived from a combined sequence of the human germline light chain IGKV1-39*01 and hjk4.1. In order to avoid a decrease in activity caused by a decrease in immunogenicity, the human antibody variable region can be subjected to minimal reverse mutation to maintain activity.

In some embodiments, the monoclonal antibody against capsid proteins of multiple AAV serotypes or the functional fragment thereof has a detectable label.

The detectable label can be selected from any one or more of a chromophore, a digoxin-labelled probe, an electron-dense substance, colloidal gold or an enzyme. These labels are listed in the non-limiting section below:
· an enzyme that produces a detectable signal, for example by colourimetry, fluorescence and luminescence, such as horseradish peroxidase, alkaline phosphatase, β-galactosidase and glucose-6-phosphate dehydrogenase;
· a chromophore, such as a fluorophore, a quantum dot, a fluorescent microsphere, a luminescent compound and a dye;
· a group having an electron density that can be detected by electron microscopy or by the electrical properties thereof, such as conductivity, amperometry, voltage measurement and resistance;
· a detectable group, for example whose molecules are of sizes which are sufficient to induce detectable modifications in the physical and/or chemical characteristics thereof, wherein this detection can be achieved by optical methods (such as diffraction, surface plasmon resonance, surface variation and angle of contact variation), or physical methods (such as atomic force spectroscopy and the tunnel effect); or
· an electron-dense substance, such as a radioactive molecule (such as ³²P, ³⁵S or ¹²⁵I).

In some embodiments, the detectable label is selected from one or more of alkaline phosphatase, acridinium ester, horseradish peroxidase, tris(bipyridine)ruthenium, isoluminol or rare earth elements, preferably alkaline phosphatase, acridinium ester or horseradish peroxidase.

In another aspect, the present invention provides an isolated polynucleotide encoding the above monoclonal antibody against capsid proteins of multiple AAV serotypes or the functional fragment thereof.

In some embodiments, the polynucleotide comprises a nucleotide sequence encoding the heavy chain variable region of the above monoclonal antibody against capsid proteins of multiple AAV serotypes or the functional fragment thereof, and a nucleotide sequence encoding the light chain variable region of the monoclonal antibody against capsid proteins of multiple AAV serotypes or the functional fragment thereof.

In another aspect, the present invention provides an expression vector comprising the polynucleotide.

The term "vector" refers to a nucleic acid delivery vehicle into which a polynucleotide can be inserted. When a vector can express the protein encoded by the inserted polynucleotide, the vector is referred to as an expression vector. The vector can be introduced into a host cell by transformation, transduction or transfection, so that the genetic material element carried by the vector is expressed in the host cell. The vector is well known to a person skilled in the art, and includes, but is not limited to: a plasmid; a phagemid; a cosmid; an artificial chromosome, e.g., a yeast artificial chromosome (YAC), a bacterial artificial chromosome (BAC) or a P1-derived artificial chromosome (PAC); a phage, such as a λ phage or an M13 phage; an animal virus; and the like. The animal virus that can be used as a vector includes, but is not limited to, a retrovirus (including a lentivirus), an adenovirus, an adeno-associated virus, a herpes virus (such as herpes simplex virus), a poxvirus, a baculovirus, a papillomavirus and a papovavirus (e.g., SV40).

In another aspect, the present invention provides a host cell or a cell-free expression system comprising the expression vector.

The host cell or cell line suitable for expressing the antigen-binding protein of the present invention includes: a mammalian cell, such as NS0, Sp2/0, CHO, COS, HEK, fibroblast and myeloma cell. Human cells can be used, thus allowing the molecule to be modified with human glycosylation patterns. Alternatively, other eukaryotic cell lines can be used. The selection of suitable mammalian host cells, and methods for transformation, culture, amplification, screening, and product production and purification are known in the art.

It can be demonstrated that bacterial cells can be used as host cells, which are suitable for expression of the recombinant Fab or other embodiments of the present invention. However, since proteins expressed in bacterial cells tend to be in an unfolded, misfolded, or non-glycosylated form, any recombinant Fab produced in bacterial cells must be screened to retain antigen-binding ability. If the molecule expressed by a bacterial cell is produced in an appropriately folded form, the bacterial cell will be the desired host. Alternatively, in an alternative embodiment, the molecule can be expressed in a bacterial host and subsequently refolded. For example, various strains of *Escherichia coli* used for expression are well-known host cells in the field of biotechnology. Various strains of *Bacillus subtilis, Streptomyces,* other *Bacillus* species, etc., can also be used in the method.

Strains of yeast cells, insect cells, such as *Drosophila* and *Lepidoptera* insects, and viral expression systems known to a person skilled in the art, can also be used as host cells, if desired.

In some embodiments, the nucleic acid is inserted into the genome of the cell and can be stably expressed.

The insertion can be achieved by using the vector as described above, or directly transferring the nucleic acid into the cell without being ligated into a vector (e.g., liposome-mediated transfection technique).

In another aspect, the present invention provides a method for preparing a monoclonal antibody against capsid proteins of multiple AAV serotypes or a functional fragment thereof, comprising:
culturing the host cell as described above under suitable culture conditions; and
recovering the antibody or the antigen-binding fragment thereof thus produced from a culture medium or from the cultured cells.

The culture method of the present invention is usually a serum-free culture method, which usually comprises serum-free suspension culture of cells. Likewise, once the antibody of the present invention is produced, the antibody can be purified from the cell culture content according to standard procedures in the art, including ammonium sulphate precipitation, affinity column, column chromatography, gel electrophoresis, etc. Such technique falls within the technical scope of the art and does not limit the present invention. Another method for expressing an antibody can be achieved by utilizing expression in animals (particularly transgenic animals or nude mice). The expression in animals involves an expression system utilizing an animal casein promoter, which, when transgenically incorporated into a mammal, allows the female animal to produce the desired recombinant protein in its milk. The culture fluid secreting the antibody can be purified by conventional techniques. For example, purification is performed using an A or G Sepharose FF column containing adjusted buffer. The purification method comprises washing away non-specifically bound components, then eluting the bound antibody by a pH gradient method, detecting antibody fragments by SDS-PAGE, and collecting same. The antibody can be filtered and concentrated by a conventional method. Soluble mixtures and polymers can also be removed by conventional methods, such as size-exclusion chromatography and ion exchange. The obtained product should be immediately frozen, such as at -70°C, or lyophilized.

In another aspect, the present invention provides a kit for detecting multiple AAV serotypes, comprising the monoclonal antibody or the functional fragment thereof as described above.

The present invention also relates to a chromatography medium for isolating multiple AAV serotypes, wherein the chromatography medium comprises a matrix support and the monoclonal antibody or the functional fragment thereof as described above immobilized on the matrix support.

In some embodiments, the matrix support includes any one of agar, agarose, an agarose derivative, a magnetic bead, silicon dioxide, titanium dioxide, alginates, cellulose, a cellulose derivative, glucan, starch, cyclodextrin, chitosan, carrageenan, guar gum, arabic gum, gum ghatti, tragacanth gum, karaya gum, locust bean gum, xanthan gum, pectin, mucin, heparin, gelatine, silicon wafer, ceramic, glass (e.g., borosilicate glass), polyurethane, polystyrene, polystyrene divinylbenzene, polymethyl methacrylate, polyacrylamide, polyethylene terephthalate, polyvinyl acetate, polyethylene, polypropylene, polyvinyl chloride, polyvinylpyrrolidone, or copolymers formed by any two or more of the above.

The matrix support can be of any shape, e.g., a substantially spherical shape and a substantially cubic shape, etc..

The present invention also relates to a chromatographic separation device containing the chromatography medium as described above.

There are many types of chromatographic separation devices, including but not limited to: SPE solid-phase extraction columns, centrifuge tubes with separation membranes, magnetic beads, separation membranes, rapid detection bio-chips, and fibre bundle columns. The chromatographic separation device is preferably a column, e.g., a monolithic column and a conventional analytical-grade or preparative-grade chromatographic column.

In another aspect, the present invention provides the use of the antibody or the antigen-binding fragment thereof as described above in the detection and/or purification of multiple AAV serotypes;
the multiple AAV serotypes include at least one of AAV1, AAV2, AAV5, AAV6, AAV8, AAV9, AAVDJ and AAVrh.1;
or the multiple AAV serotypes include at least one of AAV1, AAV2, AAV6, AAVDJ and AAVrh.1.

Beneficial effects are as follows:
current research on AAV viral delivery vectors still lacks a universal and effective serological detection method for a viral capsid protein. The monoclonal antibody against capsid proteins of AAVs developed by the present invention can specifically react with multiple AAV serotypes and can all recognize the VP1, VP2 and VP3 proteins of the virus. The provided monoclonal antibody facilitates the functional verification of AAV viral delivery vectors and the development of a detection kit thereof.

### Brief Description of the Drawings

In order to illustrate the technical solutions in the Detailed Description of Embodiments of the present invention or in the prior art more clearly, the accompanying drawings required in the description of the Detailed Description of Embodiments or the prior art are briefly described below. It is clear that the accompanying drawings in the following description illustrate some embodiments of the present invention, and those of ordinary skill in the art may further derive other drawings from these accompanying drawings without involving any inventive effort.
Fig. 1 is a graph showing the detection results of rabbit serum titres after immunization.
Fig. 2 shows the results of ELISA reactions of 5G4-1 monoclonal antibody with different serotypes of AAVs.
Fig. 3 shows the results of ELISA reactions of 24F7-1 monoclonal antibody with different serotypes of AAVs.
Fig. 4 shows the EC₅₀ results of 5G4-1 monoclonal antibody and different serotypes of AAVs.
Fig. 5 shows the EC₅₀ results of 24F7-1 monoclonal antibody and different serotypes of AAVs.
Fig. 6 shows the WB results of 5G4-1 monoclonal antibody and different serotypes of AAVs.
Fig. 7 shows the WB results of 24F7-1 monoclonal antibody and different serotypes of AAVs.

### Detailed Description of Embodiments

The present invention relates to a monoclonal antibody against capsid proteins of multiple AAV serotypes. The embodiments of the present invention will be described in detail below in combination with the Examples. Unless otherwise stated, the technical and scientific terms used in the present invention have the same meanings as commonly understood by those of ordinary skill in the art to which the present invention belongs.

The term "adeno-associated virus" (AAV) is a commonly used gene manipulation tool, which belongs to the family Parvoviridae and is a non-enveloped single-stranded linear DNA virus. The genome of AAV is about 4.7 kb in length, and the Cap gene encodes three viral capsid proteins, VP1, VP2 and VP3. There are many serotypes of AAV, including AAV1, AAV2, AAV3, AAV4, AAV5, AAV6, AAV7, AAV8, AAV9, AAVDJ, AAVrh10, etc. AAVrh.1 is an AAV virus isolated from rhesus monkey and, like other AAV viruses, is susceptible to multiple tissues and organs in the human body.

The term "virus-like particle" or "VLP" refers to a non-infectious particle that does not contain viral genomic nucleic acid and is assembled only from viral structural proteins. VLP generally has the same morphology as a mature viral particle and is highly immunogenic. VLP can be expressed in multiple expression systems, such as the mammalian expression system and the baculovirus expression system. The VLP used in the present invention are formed by the automatic assembly of VP1, VP2 and VP3 proteins of the AAV virus expressed in 293T cells.

The term "antibody" is intended to refer to an immunoglobulin molecule consisting of four polypeptide chains (two heavy chains (H) and two light chains (L) inter-connected by disulfide bonds (i.e., an "intact antibody molecule")), as well as a multimer thereof (e.g., IgM) or an antigen-binding fragment thereof. Each heavy chain is composed of a heavy chain variable region ("HCVR" or "VH") and a heavy chain constant region (composed of domains CH1, CH2 and CH3). Each light chain is composed of a light chain variable region ("LCVR" or "VL") and a light chain constant region (CL). VH and VL regions can be further subdivided into hypervariable regions called complementary determining regions (CDRs), which are interspersed with more conserved regions called framework regions (FRs). The amino acid sequences of the CDRs can be determined readily using art-recognized numbering schemes, e.g., Kabat, Chothia, IMGT, AbM or Contact. In a specific embodiment, in the present invention, the CDRs of the antibody described herein have been determined according to the Kabat numbering scheme. VH and VL each are composed of three CDRs and four FRs, which are arranged from an amino terminus to a carboxyl terminus in the following order: FR1, CDR1, FR2, CDR2, FR3, CDR3 and FR4. In some embodiments of the present invention, the FRs of the antibody (or the antigen-binding fragment thereof) may be identical to human germline sequences or may be modified naturally or artificially.

The term "monoclonal antibody" refers to a homogeneous antibody that targets only a specific antigenic epitope. Compared with common polyclonal antibody preparations which typically comprise different antibodies targeting different antigenic determinants (epitopes), each monoclonal antibody targets a single antigenic determinant on an antigen. The modifier "monoclonal" indicates the homogeneous characteristic of an antibody and is not to be construed as requiring production of the antibody by any particular method. The monoclonal antibody of the present invention is preferably produced by a recombinant DNA method or obtained by a screening method described elsewhere herein.

The term "mutation" means that a polypeptide comprises alterations, i.e., substitutions, insertions and/or deletions, in one or more (several) amino acid residues at one or more (several) positions on a monoclonal antibody or a functional fragment thereof. A substitution refers to replacing an amino acid occupying a position with a different amino acid; a deletion refers to removing an amino acid occupying a position; and an insertion refers to adding 1-3 amino acids immediately following an amino acid occupying a position.

The term "isolated polynucleotide" refers to a polynucleotide that does not occur naturally in nature, including polynucleotides isolated from nature (including organisms) by biological techniques, and polynucleotides artificially synthesized. The isolated polynucleotide can be genomic DNA, cDNA, mRNA or other synthetic RNA, or a combination thereof. It should be noted that according to the amino acid sequences of the heavy chain variable region and light chain variable region provided herein, based on codon degeneracy, a person skilled in the art can design a nucleotide sequence that is not completely identical to the provided nucleotide sequence but encode the same amino acid sequence. These altered nucleotide sequences are also included within the scope of the present invention.

When referring to a polynucleotide, the term "vector" refers to any molecule (e.g., a nucleic acid, a plasmid or a virus) used to transfer nucleotide encoding information into a host cell. The term "expression vector" or "expression cassette" refers to a vector suitable for expression of a target gene (a nucleotide sequence to be expressed) in a host cell, and usually includes a target gene, a promoter, a terminator, a marker gene, etc.

The term "host cell" refers to a cell that has been or is capable of being transformed with a nucleic acid sequence and thereby expresses the selected target gene. The term includes the progeny of a parent cell, regardless of whether the progeny is identical in morphology or genetic composition to the original parent cell, as long as the selected target gene is present in the progeny. Common host cells include a bacterium, a yeast, a mammalian cell, etc.

The term "antibody functional fragment" means an antigen-binding fragment of an antibody and an antibody analogue, which usually include at least a portion of the antigen-binding region or variable region (e.g., one or more CDRs) of the parental antibody. The antibody fragment retains at least some of the binding specificity of the parental antibody. For example, antibody fragments capable of binding to an AAV viral capsid protein or a portion thereof include, but are not limited to, sdAb (a single-domain antibody), Fab (e.g., obtained by papain digestion of an antibody), F(ab')2 (e.g., obtained by pepsin digestion), Fv or scFv (e.g., obtained by molecular biology techniques).

The term "amino acid substitution" refers to replacing an existing amino acid residue in a predetermined (initial) amino acid sequence with a different amino acid residue. In general, it is recognized by a person skilled in the art that a single amino acid substitution in a non-essential region of a polypeptide does not substantially alter the biological activity (see, e.g., Watson et al., Molecular Biology of the Gene, The Benjamin/Cummings Pub. Co., p. 224 (Fourth Edition, 1987)). Such exemplary substitutions are preferably performed according to the substitutions shown below:

**Table 1 Exemplary conservative amino acid substitutions**

| Original residue | Conservative substitution |
|---|---|
| Ala (A) | Gly; Ser |
| Arg (R) | Lys; His |
| Asn (N) | Gln; His |
| Asp (D) | Glu; Asn |
| Cys (C) | Ser; Ala |
| Gln (Q) | Asn |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |

The "percent (%) amino acid sequence identity" with respect to a peptide or polypeptide sequence is defined as the percentage of amino acid residues in a candidate sequence that are identical to the amino acid residues in the specific peptide or polypeptide sequence, after aligning the sequences and introducing gaps, if necessary, to achieve the maximum percent sequence identity, and not considering any conservative substitutions as part of the sequence identity. Alignment of sequences for the purpose of determining the percent amino acid sequence identity can be achieved by using a variety of methods within the technical scope of the art, for example, using publicly available computer software, such as BLAST, BLAST-2, ALIGN or Megalign (DNASTAR) software. A person skilled in the art can determine appropriate parameters for measuring alignment, comprising any algorithms needed to achieve maximal alignment over the full length of the sequences being compared.

Unless specifically stated otherwise, the singular includes the plural. Unless specifically stated otherwise, the word "a" or "an" means "at least one". Unless otherwise stated, "or" means "and/or". The phrase "at least one" is equivalent in meaning to the phrase "one or more". Further, the term "including" and other forms such as "includes" and "included" are used in a non-limiting manner. Further, unless specifically stated otherwise, terms such as "element" or "component" include an element or component comprising one unit and an element and component comprising more than one unit.

Unless otherwise specified, the methods and materials in the examples described below are conventional products that can be purchased from the market. A person skilled in the art of the present invention would understand that the methods and materials described below are exemplary only and should not be considered as limiting the scope of the present invention.

### Example 1: Immunization of animals with rh.1-type AAV virus-like particles

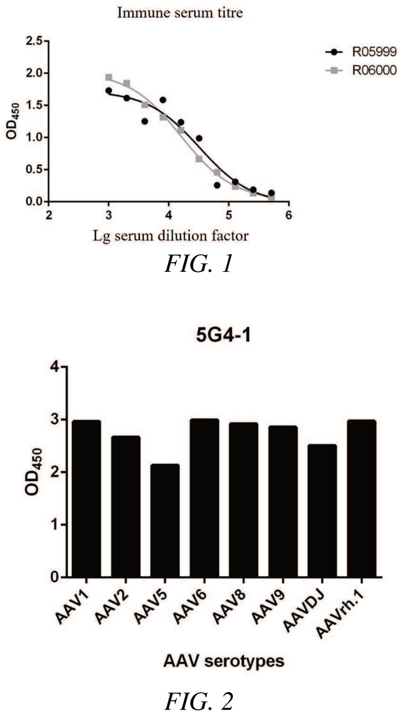

New Zealand white rabbits were immunized with 100 µg of recombinant rh.1-type AAV virus-like particles (AAVrh.1 VLP, the protein sequences of the capsid proteins VP1, VP2 and VP3 were as set forth in SEQ ID NOs: 1, 2 and 3). Subsequently, the immunization was repeated every other week to boost the immunity to the experimental rabbits for a total of three times. Two rabbits had serum titres of 10⁵ or more after three immunizations (Fig. 1). 7 days after the last immunization, sterile blood was collected from the experimental rabbit numbered R05999 for subsequent antibody discovery work.

Amino acid sequence of capsid protein VP1 of rh.1-serotype AAV (SEQ ID NO: 1):

MAADGYLPDWLEDNLSEGIREWWALKPGAPKPKANQQKQDDGRGL
VLPGYKYLGPFNGLDKGEPVNAADAAALEHGKAYDQQLQAGDNPYLRY
NHADAEFQERLQEDTSFGGNLGRAVFQAKKRVLEPLGLVEEGAKTAPGK
KRPVEPSPQRSPDSSTGIGKKGQQPARKRLNFGQTGDSESVPDPQPLGEPPA
APSGVGPNTMAAGGGAPMADNNEGADGVGNSSGNWHCDSTWLGDRVIT
TSTRTWALPTYNNHLYKQISNGTSGGSTNDNTYFGYSTPWGYFDFNRFHC
HFSPRDWQRLINNNWGFRPKRLNFKLFNIQVKEVTTNEGTKTIANNLTSTV
QVFTDSEYQLPYVLGSAHQGCLPPFPADVFMVPQYGYLTLNNGSQALGRS
SFYCLEYFPSQMLRTGNNFQFSYTFEDVPFHSSYAHSQSLDRLMNPLIDQY
LYYLVRTQTTGTGGTQTLAFSQAGPSSMANQARNWVPGPCYRQQRVSTT
TNQNNNSNFAWTGAAKFKLNGRDSLMNPGVAMASHKDDEDRFFPSSGVL
IFGKQGAGNDGVDYSQVLITDEEEIKATNPVATEEYGAVAINNQAANTQA
QTGLVHNQGVIPGMVWQNRDVYLQGPIWAKIPHTDGNFHPSPLMGGFGL
KHPPPQILIKNTPVPADPPLTFNQAKLNSFITQYSTGQVSVEIEWELQKENS
KRWNPEIQYTSNYYKSTNVDFAVNTKGVYSEPRPIGTRYLTRNL

Amino acid sequence of capsid protein VP2 of rh.1-serotype AAV (SEQ ID NO: 2):

MAPGKKRPVEPSPQRSPDSSTGIGKKGQQPARKRLNFGQTGDSESVPD
PQPLGEPPAAPSGVGPNTMAAGGGAPMADNNEGADGVGNSSGNWHCDS
TWLGDRVITTSTRTWALPTYNNHLYKQISNGTSGGSTNDNTYFGYSTPWG
YFDFNRFHCHFSPRDWQRLINNNWGFRPKRLNFKLFNIQVKEVTTNEGTK
TIANNLTSTVQVFTDSEYQLPYVLGSAHQGCLPPFPADVFMVPQYGYLTLN
NGSQALGRSSFYCLEYFPSQMLRTGNNFQFSYTFEDVPFHSSYAHSQSLDR
LMNPLIDQYLYYLVRTQTTGTGGTQTLAFSQAGPSSMANQARNWVPGPC

YRQQRVSTTTNQNNNSNFAWTGAAKFKLNGRDSLMNPGVAMASHKDDE
DRFFPSSGVLIFGKQGAGNDGVDYSQVLITDEEEIKATNPVATEEYGAVAI
NNQAANTQAQTGLVHNQGVIPGMVWQNRDVYLQGPIWAKIPHTDGNFHP
SPLMGGFGLKHPPPQILIKNTPVPADPPLTFNQAKLNSFITQYSTGQVSVEIE
WELQKENSKRWNPEIQYTSNYYKSTNVDFAVNTKGVYSEPRPIGTRYLTR
NL

Amino acid sequence of capsid protein VP3 of rh.1-serotype AAV (SEQ ID NO: 3):

MAAGGGAPMADNNEGADGVGNSSGNWHCDSTWLGDRVITTSTRTW
ALPTYNNHLYKQISNGTSGGSTNDNTYFGYSTPWGYFDFNRFHCHFSPRD
WQRLINNNWGFRPKRLNFKLFNIQVKEVTTNEGTKTIANNLTSTVQVFTDS
EYQLPYVLGSAHQGCLPPFPADVFMVPQYGYLTLNNGSQALGRSSFYCLE
YFPSQMLRTGNNFQFSYTFEDVPFHSSYAHSQSLDRLMNPLIDQYLYYLVR
TQTTGTGGTQTLAFSQAGPSSMANQARNWVPGPCYRQQRVSTTTNQNNN
SNFAWTGAAKFKLNGRDSLMNPGVAMASHKDDEDRFFPSSGVLIFGKQG
AGNDGVDYSQVLITDEEEIKATNPVATEEYGAVAINNQAANTQAQTGLVH
NQGVIPGMVWQNRDVYLQGPIWAKIPHTDGNFHPSPLMGGFGLKHPPPQI
LIKNTPVPADPPLTFNQAKLNSFITQYSTGQVSVEIEWELQKENSKRWNPEI
QYTSNYYKSTNVDFAVNTKGVYSEPRPIGTRYLTRNL

### Example 2: Acquisition of B cells and screening of monoclonal antibody

1) Isolation of antigen-positive B cells
   15 mL of anticoagulated rabbit blood was aseptically collected for PBMC isolation, and then antigen-positive cells were enriched using an antigen. The enriched cells were plated at a density of 10 cells/well into a 96-well cell culture plate pre-plated with feeder cells. The plate was incubated at 37°C in 5% CO₂. On day 7, the supernatant of the overnight culture was collected and detected for the presence of the antibody against an AAV virus-like particle using the indirect ELISA method described below.
2) Indirect ELISA

Indirect ELISA was used to assess the binding ability of the antibody in the supernatant to VLP. AAVrh.1 VLPs were diluted to 1 µg/mL with PBS, and then 96-well ELISA plates were coated with the dilution at 100 µL/well overnight at 4°C. The plates were washed with PBST (0.05% Tween), and then blocked with PBST containing 1% BSA at 150 µL/well for 1 hour at 37°C. Subsequently, the blocking solution was discarded, 100 µL of the B cell culture supernatant was added to each plate, and the plates were then incubated for 1 hour at 37°C. The supernatant was discarded and the plates were washed three times with PBST. Then, the plates were incubated with the horseradish peroxidase-labelled goat anti-rabbit IgG (Fc-specific) secondary antibody (GenScript, A01856) at 100 µL/well for 0.5 hours at 37°C. The plates were washed 5 times with PBST, a TMB colour developing solution was then added, and the mixture was reacted for 13 minutes at room temperature in the dark. Finally, 50 µL of the 1 M HCl stop solution (Sinopharm, 10011018) was added to stop colour development. The plates were read at 450 nm using a microplate reader. The cells in the positive wells with absorbance values greater than 1.0 were selected for subsequent experiments.

### Example 3: Sequencing of variable regions of monoclonal antibody

RNA from total cells in wells with OD values greater than 1.0 was extracted using TRIzol (Life Technology, 15596-026), and reverse-transcribed into cDNA using universal primers (Prime Script^{™} 1^{st} Strand cDNA Synthesis Kit, Takara). Subsequently, heavy chain and light chain V-region fragments of rabbit immunoglobulin were amplified by RACE PCR, the amplified fragments were inserted into the pCDNA3.4 vector by homologous recombination, and the inserted fragments were sequenced using vector-specific primers. Finally, the unique V-region protein amino acid sequences and the plasmids of clones 5G4-1 and 24F7-1 were obtained.

Amino acid sequence of heavy chain variable region of 5G4-1 (SEQ ID NO: 4):

QSVKESEGGLFKPADTLSLTCTVSGFSLNNNDMSWVRQAPGNGLEYI
GVMSSGGRLYAATWAKSRFTITRNTNLGTVTLKLTSLTAADTATYFCARG
SPSSNRGIDIWGPGTLVTVSS

Amino acid sequence of light chain variable region of 5G4-1 (SEQ ID NO: 5):

LTQTPSSVSAPVGGTVSINCQSDKSVYADNYLSWYQQTPGQPPKLLIY
KASTLASGIPSRFKGSGSGTQFTLAISDVQCDDAATYYCAGGYSGNSDNGF
GGGTEVVVR

Amino acid sequence of heavy chain variable region of 24F7-1 (SEQ ID NO: 6):

QSLRESEGGLFKPADTLTLTCTVSGFSITAYNVGWVRQAPGKGLEWIG
VIDISGATFYATWAKSRSTITRDTSLNTVTLKMTSLTAADTATYFCTRDPAF
IWGPGTLVTVSS

Amino acid sequence of light chain variable region of 24F7-1 (SEQ ID NO: 7):

DVVMTQTPASVEAAVGGTVTIKCQASQSVSSYLAWYQQKPGQPPKL
LIYRASTLASGVPSRFKGSGSGTEFTLTISDLECADAATYYCQSYVYSNVLT
YFNAFGGGTGVVVK

**Table 2. CDR region sequences of antibodies**

| Antibody number | CDR1/sequence number | CDR2/sequence number | CDR3/sequence number |
|---|---|---|---|
| 5G4-1 V_{H} | NNDMS (SEQ ID NO:8) | VMSSGGRLYAATWAKS (SEQ ID NO:9) | GSPSSNRGIDI (SEQ ID NO:10) |
| 5G4-1 V_{L} | QSDKSVYADNYLS (SEQ ID NO:11) | KASTLAS (SEQ ID NO:12) | AGGYSGNSDNG (SEQ ID NO:13) |
| 24F7-1 V_{H} | AYNVG (SEQ ID NO:14) | VIDISGATFYATWAKS (SEQ ID NO:15) | DPAFI (SEQ ID NO:16) |
| 24F7-1 V_{L} | QASQSVSSYLA (SEQ ID NO:17) | RASTLAS (SEQ ID NO:18) | QSYVYSNVLTYFNA (SEQ ID NO:19) |

### Example 4: Production of monoclonal antibody based on recombinant expression

Plasmids containing antibody heavy and light chains, respectively, were co-transfected into ExpiCHO-S^{™} cells (Gibco, A29133), the cells were cultured in a shake flask at 37°C for 6 days, and then the supernatant was collected for antibody purification. Antibody purification steps: A protein A column was equilibrated with a buffer containing 0.05 M Tris and 1.5 M NaCl (pH 8.0). Subsequently, the harvested cell culture supernatant was diluted at 1 : 1 with 2× the above buffer and sterilized by filtration. The filtered supernatant and protein A column were incubated for 2 hours at room temperature. The column was washed with 1× the above buffer, IgG was then eluted with sterile 0.1 M sodium citrate (pH 3.5), and the eluate was collected and neutralized with one-ninth volume of sterile 1 M Tris-HCl (pH 9.0). Under sterile conditions, the product buffer was exchanged to PBS (pH 7.4) to remove the residual elution buffer, and the antibody was quantified by OD280 nm using an extinction coefficient Ec of 1.43 (0.1%).

### Example 5: Cross-reactivity of monoclonal antibody to VLPs of multiple serotypes of AAV viruses

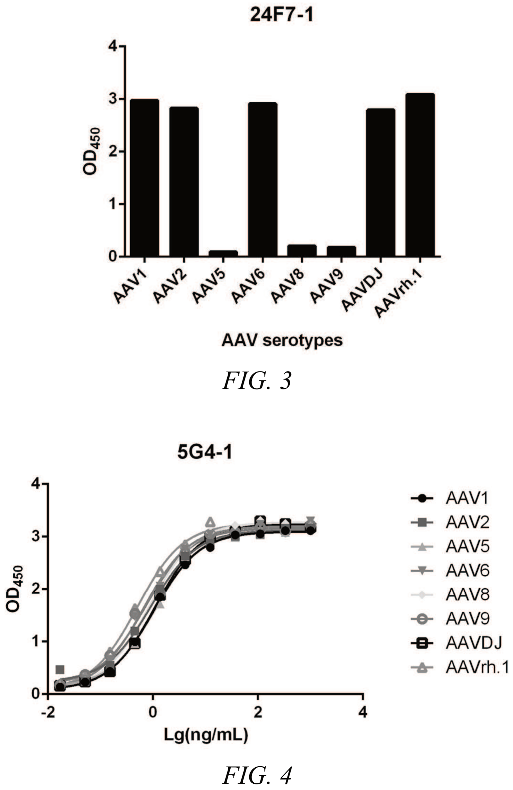

Indirect ELISA was used to assess the binding ability of the purified antibody to VLPs of AAV1, AAV2, AAV5, AAV6, AAV8, AAV9, AAVDJ and AAVrh.1. 100 µL of 1 µg/mL VLP diluted in PBS was added to each well of 96-well ELISA plates, and the plates were coated overnight at 4°C. The plates were washed with PBS-T (0.05% Tween), and then blocked with PBST containing 1% BSA at 150 µL/well for 1 hour at 37°C. Subsequently, the blocking solution was discarded, 100 µL of recombinant expressed antibody at a concentration of 1 µg/mL was added to each plate, and the plates were then incubated for 1 hour at 37°C. The plates were washed three times with PBST, 100 µL of the horseradish peroxidase-labelled goat anti-rabbit IgG (Fc-specific) secondary antibody (GenScript, A01856) was then added to each well, and the plates were incubated for 0.5 hours at 37°C. The plates were washed five times with PBST, a TMB colour developing solution was then added, and the plates were incubated for 13 minutes at room temperature in the dark. Finally, 50 µL of the 1 M HCl stop solution (Sinopharm, 10011018) was added to stop colour development. The plates were read at 450 nm using a microplate reader, and the results were as shown in Fig. 2 and Fig. 3. The antigen-antibody combinations with absorbance values greater than 0.3 were selected for subsequent EC₅₀ experiments.

### Example 6: EC₅₀ test of monoclonal antibody binding to VLPs of multiple serotypes of AAV viruses

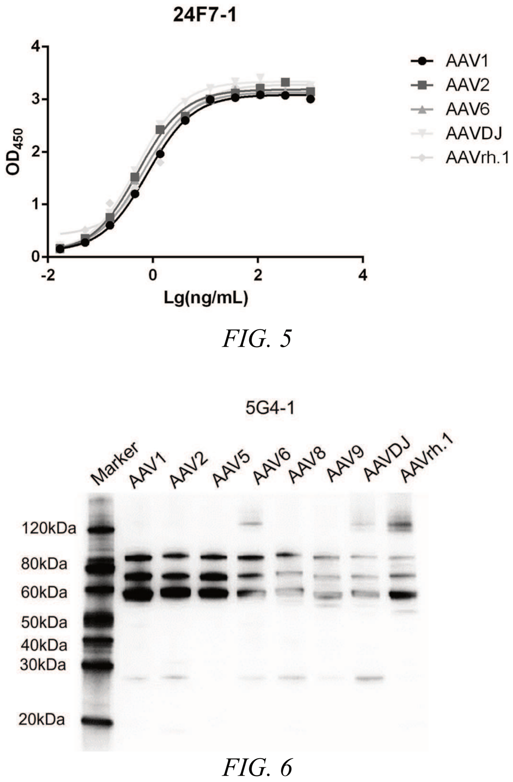

Indirect ELISA was used to assess the binding ability of the purified antibody to VLPs of multiple serotypes of AAV viruses. 96-well ELISA plates were coated with 1 µg/mL of VLPs of each serotype of AAV overnight at 4°C. The plates were washed with PBS-T (0.05% Tween), and then blocked with PBST containing 1% BSA at 250 µL/well for 2 hours at 37°C. Subsequently, the blocking solution was discarded, and 100 µL of the 1 µg/mL purified antibody was added to the first well and subjected to 3-fold serial dilution to obtain a total of 11 test concentration gradients. Additionally, one control well containing only PBST was prepared. Then, the plates were incubated for 1 hour at 37°C. The plates were washed three times with PBST, 100 µL of the horseradish peroxidase-labelled goat anti-rabbit IgG (Fc-specific) secondary antibody (GenScript, A01856) was then added to each well, and the plates were incubated for 0.5 hours at 37°C. The plates were washed four times with PBST, a TMB colour developing solution (GenScript) was then added, and the plates were incubated for 15 minutes at room temperature in the dark. Finally, 50 µL of the 1 M HCl stop solution (Sinopharm, 10011018) was added to stop colour development. The plates were read at 450 nm using a microplate reader. The EC₅₀ detection curve for each monoclonal antibody was as shown in Fig. 4 and Fig. 5, and the EC₅₀ values were as shown in Table 3.

**Table 3: EC₅₀ values of antibody binding to VLPs of multiple serotypes of AAV viruses**

| Antibody | Antigen | EC₅₀ (ng/ml) |
|---|---|---|
| 5G4-1 | AAV1 | 1.03 |
| | AAV2 | 0.94 |
| | AAV5 | 1.16 |
| | AAV6 | 0.64 |
| | AAV8 | 1.00 |
| | AAV9 | 0.64 |
| | AAVDJ | 1.12 |
| | AAVrh.1 | 0.51 |
| 24F7-1 | AAV1 | 0.79 |
| | AAV2 | 0.51 |
| | AAV6 | 0.61 |
| | AAVDJ | 0.50 |
| | AAVrh.1 | 0.93 |

### Example 7: Cross-reactivity of monoclonal antibody to VLPs of multiple serotypes of AAV viruses detected by WB

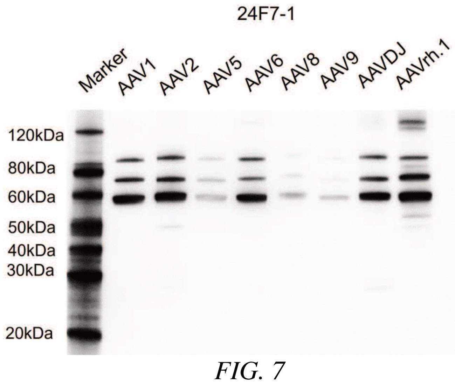

WB was used to evaluate the reactivity of a monoclonal antibody to three capsid proteins VP1, VP2 and VP3 of different serotypes of AAV viruses. VLPs of AAV1, AAV2, AAV5, AAV6, AAV8, AAV9, AAVDJ and AAVrh.1 were diluted to 0.015 mg/mL, each sample was mixed with reducing 2× loading buffer at a ratio of 1 : 1, and then the mixture was placed in a boiling water bath for 5 min. 10 µL of the treated samples were respectively added to sample wells with 10% precast gel (GenScript, M42012C) for SDS-PAGE, followed by transfer to an NC membrane. The membrane was blocked with 5% skim milk diluted in PBST at 37°C for 2 h. The solution was replaced with 0.1 µg/mL of the monoclonal antibody diluted in skim milk, and incubation was performed for 1 h. The membrane was washed five times with PBST, and then incubated with the horseradish peroxidase-labelled goat anti-rabbit IgG (Fc-specific) secondary antibody (GenScript, A01856) for 1 hour at 37°C. The membrane was washed five times with PBST, a chemiluminescent solution was added, and then the membrane was imaged on the ChemiDoc MP chemiluminescent imaging system (Bio-Rad). The results of WB reactions of each monoclonal antibody against different serotypes of AAV virus were as shown in Fig 6 and Fig 7.

The above examples merely represent several embodiments of the present invention, giving specifics and details thereof, but should not be understood as limiting the scope of the present patent of invention thereby. It should be noted that those of ordinary skill in the art could also make several alterations and improvements without departing from the spirit of the present invention and these would all fall within the scope of protection of the present invention. Therefore, the scope of protection of the present patent of invention shall be in accordance with the appended claims, and the description and accompanying drawings can be used to explain the content of the claims.

## Claims

1. A monoclonal antibody against capsid proteins of multiple AAV serotypes or a functional fragment thereof, **characterized in that** the antibody or the functional fragment thereof comprises heavy chain CDRs and light chain CDRs, the heavy chain CDRs and light chain CDRs are selected from the group consisting of CDRs with amino acid sequences as set forth in SEQ ID NO: 8 to SEQ ID NO: 19 and variants thereof each comprising at most three amino acid mutations, and the group is defined in terms of a combination pattern by a and/or b:
| Combination name | Heavy chain CDR1 | Heavy chain CDR2 | Heavy chain CDR3 | Light chain CDR1 | Light chain CDR2 | Light chain CDR3 |
|---|---|---|---|---|---|---|
| a | SEQ ID NO:8 or a variant thereof | SEQ ID NO:9 or a variant thereof | SEQ ID NO:10 or a variant thereof | SEQ ID NO:11 or a variant thereof | SEQ ID NO: 12 or a variant thereof | SEQ ID NO: 13 or a variant thereof |
| b | SEQ ID NO: 14 or a variant thereof | SEQ ID NO: 15 or a variant thereof | SEQ ID NO: 16 or a variant thereof | SEQ ID NO: 17 or a variant thereof | SEQ ID NO: 18 or a variant thereof | SEQ ID NO: 19 or a variant thereof |

2. The monoclonal antibody against capsid proteins of multiple AAV serotypes or the functional fragment thereof according to claim 1, **characterized in that** the heavy chain CDRs and light chain CDRs are selected from the following sequences:
a. heavy chain CDR1, heavy chain CDR2, heavy chain CDR3, light chain CDR1, light chain CDR2, and light chain CDR3 with amino acid sequences as set forth in SEQ ID NOs: 8, 9, 10, 11, 12 and 13, respectively; or
b. heavy chain CDR1, heavy chain CDR2, heavy chain CDR3, light chain CDR1, light chain CDR2, and light chain CDR3 with amino acid sequences as set forth in SEQ ID NOs: 14, 15, 16, 17, 18 and 19, respectively.

3. The monoclonal antibody against capsid proteins of multiple AAV serotypes or the functional fragment thereof according to claim 1 or 2, **characterized by** comprising a heavy chain variable region and a light chain variable region, wherein the heavy chain variable region and light chain variable region are selected from the group consisting of variable regions with amino acid sequences as set forth in SEQ ID NO: 4 to SEQ ID NO: 7 and variants thereof each comprising an amino acid sequence having at least 80% identity thereto, and the group is defined in terms of a combination pattern by A and/or B:
| Combination name | Heavy chain variable region | Light chain variable region |
|---|---|---|
| A | SEQ ID NO: 4 or a variant thereof | SEQ ID NO: 5 or a variant thereof |
| B | SEQ ID NO: 6 or a variant thereof | SEQ ID NO: 7 or a variant thereof |

4. The monoclonal antibody against capsid proteins of multiple AAV serotypes or the functional fragment thereof according to claim 3, **characterized in that** the heavy chain variable region and light chain variable region are selected from the following sequences:
A. the heavy chain variable region comprising an amino acid sequence as set forth in SEQ ID NO: 4 and the light chain variable region comprising an amino acid sequence as set forth in SEQ ID NO: 5; or
B. the heavy chain variable region comprising an amino acid sequence as set forth in SEQ ID NO: 6 and the light chain variable region comprising an amino acid sequence as set forth in SEQ ID NO: 7.

5. The monoclonal antibody against capsid proteins of multiple AAV serotypes or the functional fragment thereof according to any one of claims 1, 2 and 4, **characterized in that** the monoclonal antibody against capsid proteins of multiple AAV serotypes or the functional fragment thereof is a rabbit-derived antibody, a chimeric antibody, a humanized antibody or a human antibody.

6. The monoclonal antibody against capsid proteins of multiple AAV serotypes or the functional fragment thereof according to any one of claims 1, 2 and 4, **characterized in that** the monoclonal antibody against capsid proteins of multiple AAV serotypes or the functional fragment thereof has a detectable label.

7. An isolated polynucleotide, **characterized by** encoding the monoclonal antibody against capsid proteins of multiple AAV serotypes or the functional fragment thereof according to any one of claims 1-6.

8. The polynucleotide according to claim 7, **characterized in that** the polynucleotide comprises a nucleotide sequence encoding the heavy chain variable region of the monoclonal antibody or the functional fragment thereof, and a nucleotide sequence encoding the light chain variable region of the monoclonal antibody or the functional fragment thereof.

9. An expression vector, **characterized by** comprising the polynucleotide according to claim 7 or 8.

10. A host cell or a cell-free expression system, **characterized by** comprising the expression vector according to claim 9.

11. A method for preparing a monoclonal antibody against multiple AAV serotypes or a functional fragment thereof, **characterized by** comprising:
culturing the host cell according to claim 10 under suitable culture conditions; and
recovering the antibody or the antigen-binding fragment thereof thus produced from a culture medium or from the cultured cells.

12. A kit for detecting multiple AAV serotypes, **characterized by** comprising the monoclonal antibody or the functional fragment thereof according to any one of claims 1-6.

13. A chromatography medium for isolating multiple AAV serotypes, **characterized in that** the chromatography medium comprises a matrix support and the monoclonal antibody or the functional fragment thereof according to any one of claims 1-6 immobilized on the matrix support.

14. A chromatographic separation device, **characterized in that** the chromatographic separation device contains the chromatography medium according to claim 13.

15. Use of the antibody or the functional fragment thereof according to any one of claims 1-6 in the detection and/or purification of multiple AAV serotypes;
**characterized in that** the multiple AAV serotypes include at least one of AAV1, AAV2, AAV5, AAV6, AAV8, AAV9, AAVDJ and AAVrh.1; or the multiple AAV serotypes include at least one of AAV1, AAV2, AAV6, AAVDJ and AAVrh.1.
